Europäisches Patentamt

European Patent Office

Office européen des brevets

(19)

(11) Veröffentlichungsnummer: **0 270 782 B1**

(12) **EUROPÄISCHE PATENTSCHRIFT**

(45) Veröffentlichungstag der Patentschrift: **12.08.92**

(51) Int. Cl.⁵: **A61K 31/275**

(21) Anmeldenummer: **87115378.9**

(22) Anmeldetag: **21.10.87**

(54) **Wirkstoffe zur Verhütung von Tumormetastasen.**

(30) Priorität: **22.10.86 DE 3635931**

(43) Veröffentlichungstag der Anmeldung:
**15.06.88 Patentblatt 88/24**

(45) Bekanntmachung des Hinweises auf die
Patenterteilung:
**12.08.92 Patentblatt 92/33**

(84) Benannte Vertragsstaaten:
**AT BE CH DE ES FR GB IT LI NL SE**

(56) Entgegenhaltungen:
**EP-A- 0 159 678**
**DE-A- 2 059 985**

**Cancer chemother. pharmac., Band 14, 1985,
Seiten 30-33 Springer Verlag, Berlin, DE T.
TSURUO et al.:Inhibition of spontaneous and
experimental tumor metastasis by the calcium antagonist verapamil"**

**A. GOODMAN and GILMAN'S THE PHARMA-
COLOGICAL BASIS OF THERAPEUTICS, 7.
Auflage, 1985. Seiten 44-48, Macmillan Publishing Company, New York, US**

(73) Patentinhaber: **BASF Aktiengesellschaft
Carl-Bosch-Strasse 38
W-6700 Ludwigshafen(DE)**

(72) Erfinder: **Seitz, Werner, Dr.
Bismarckstrasse 22B
W-6831 Plankstadt(DE)**
Erfinder: **Daum, Lothar, Dr.
Reiherstrasse 25
W-6701 Otterstadt(DE)**
Erfinder: **Emling, Franz, Dr.
Valentin-Bauer-Strasse 22
W-6700 Ludwigshafen(DE)**
Erfinder: **Keilhauer, Gerhard, Dr.
Industriestrasse 20
W-6701 Dannstadt-Schauernheim(DE)**

## Beschreibung

In der DE-PS 1 154 810 sind basisch substituierte Phenylacetonitrile beschrieben. Aus dieser Verbindungsklasse haben sich aufgrund ihrer calciumantagonistischen Wirkung in der Therapie der koronaren Herzkrankheit und des Bluthochdrucks das Verapamil und das Gallopamil bewährt. Beide Verbindungen werden in der Therapie in racemischer Form eingesetzt. In der DE-OS 2 059 923 sind die linksdrehenden Antipoden von Verapamil und Gallopamil beschrieben. Beide Verbindungen sind dem Racemat in der Coronarwirksamkeit deutlich überlegen. In der DE-PS 20 59 985 sind die rechtsdrehenden Antipoden von Verapamil und Gallopamil offenbart. Der Abstand zwischen antiarrhythmischer Wirkung und negativ inotroper Wirkung ist bei den rechtsdrehenden Antipoden wesentlich günstiger.

In der EP-OS 147 707 sind die Antipoden von Emopamil und ihre Verwendung zur protektiven Behandlung von hypoxischen Gewebsschädigungen beschrieben. Beide Enantiomere zeigen dosisabhängig protektive Wirkung. Die Wirkdosis der linksdrehenden Antipoden liegt um den Faktor 8 bis 10 niedriger als die des rechtsdrehenden Antipoden.

Die deutliche Überlegenheit der linksdrehenden Antipoden von Verapamil, Gallopamil und Devapamil hinsichtlich der cardialen Wirkung ist auch der Arbeit von H. Nawrath und M. Raschack (Cell. Calcium 5, 316 (1984)) zu entnehmen. Die linksdrehenden Antipoden sind in ihrer calciumantigonistischen Wirkung bis zum Faktor 200 den rechtsdrehenden Antipoden überlegen. In der negativen inotropen Wirkung wurde ein Abstand bis zum Faktor 90 gefunden.

Weiter ist in der EP-OS 159 678 und in der Publikation von T. Tsuruo u.a. (Cancer Chemother. Pharmacol. 14, 30 (1985)) die Verwendung von racemischem Verapamil zur Behandlung von Tumormetastasen beschrieben. Die dort applizierten Dosen sind therapeutisch nur beschränkt bzw. nicht anwendbar, da die kardiale Eigenwirkung von Verapamil derartig hohe Dosen an Menschen in der Praxis ausschließt.

Überraschenderweise wurde nun gefunden, daß sich die beiden enantiomeren Formen von Verapamil, Gallopamil, Devapamil und Emopamil in der Inhibierung von spontanen und experimentellen Tumormetastasen nicht unterscheiden. Da die kardiale Wirkung der Racemate überwiegend durch die linksdrehenden Antipoden zustande kommt, bietet die Verwendung der rechtsdrehenden Enantiomeren die Möglichkeit, in ausreichend hohen Dosen zu applizieren bei gleichzeitiger Minimierung der kardialen Eigenwirkung. Dies stellt eine wesentliche Verbesserung des therapeutischen Indexes dar.

Gegenstand der Erfindung ist die Verwendung von (+)-Verapamil, (+)-Gallopamil, (+)-Devapamil und/oder (+)-Emopamil zur Herstellung von Arzneimitteln zur Verhütung von Tumormetastasen.

Verapamil ist 5-[(3,4-Dimethoxyphenethyl)-methylamino]-2-isopropyl-2-(3,4-dimethoxyphenyl)-valeronitril,
Gallopamil ist 5-[(3,4-Dimethoxyphenethyl)-methylamino]-2-isopropyl-2-(3,4,5-trimethoxyphenyl)-valeronitril,
Devapamil ist 5-[(3-Methoxyphenethyl)-methylamino]-2-isopropyl-2-(3,4-dimethoxyphenyl)-valeronitril,
Emopamil ist 5-[(Phenethyl)methylamino]-2-isopropyl-2-phenyl-valeronitril,
Die oben genannten Substanzen können gewünschtenfalls in Form ihrer Salze mit physiologisch Säuren vorliegen. Als physiologisch verträgliche Säuren kommen vorzugsweise in Betracht: Salzsäure, Schwefelsäure, Phosphorsäure, Essigsäure, Zitronensäure, Malonsäure, Salicylsäure, Maleinsäure, Fumarsäure, Bernsteinsäure, Ascorbinsäure, Äpfelsäure, Methansulfonsäure, Milchsäure, Gluconsäure, Glucuronsäure, Amidosulfonsäure, Benzoesäure, Weinsäure.

Die Dosierung der genannten Substanzen als Antimetastasen-Mittel ist je nach Art des Krebses und des Krankheitszustands verschieden. In der Regel beträgt die tägliche Dosis beim Erwachsenen pro Tag 300 bis 1000 mg bei oraler Gabe, bei intravenöser Gabe 200 bis 300 mg und bei intraperitonealer Gabe 200 bis 500 mg.

Die Substanzen können in Form von Tabletten, Kapseln oder Dragees zur oralen Applikation oder als Injektionslösung zur parenteralen (i.v., i.p. i.m.) Applikation vorliegen. Lösungen können auch infundiert werden. Die Herstellung der Applikationsformen geschieht in bekannter Weise nach üblichen Methoden.

Die Substanzen können auch Patienten verabfolgt werden, die bereits anderen Tumortherapien - z.B. Chemotherapie, Endokrintherapie, Immuntherapie, Bestrahlung oder Chirurgie - unterworfen worden sind oder unterworfen werden.

Beispiel

Es werden in üblicher Weise Oblongtabletten folgender Zusammensetzung hergestellt:

500 mg (+)-Verapamil
120 mg Lactose
60 mg Cellulose
3 mg Magnesiumstearat
50 mg Maisstärke
15 mg Polyvinylpyrrolidon.

## Patentansprüche

1. Verwendung von (+)-Verapamil, (+)-Gallopamil, (+)-Devapamil und/oder (+)-Emopamil zur Herstellung von Arzneimitteln zur Verhütung von Tumormetastasen.

**Claims**

1. Use of (+)-verapamil, (+)-gallopamil, (+)-devapamil and/or (+)-emopamil for the preparation of drugs for the prevention of tumor metastases.

**Revendications**

1. Utilisation de (+)-Verapamil, (+)-Gallopamil, (+)-Devapamil et/ou (+)-Emopamil pour la préparation de médicaments pour le traitement de métastases de tumeurs.